(19) Europäisches Patentamt
European Patent Office
Office européen des brevets

(11) Numéro de publication : **0 335 115 B1**

(12) # FASCICULE DE BREVET EUROPEEN

(45) Date de publication du fascicule du brevet : **06.11.91 Bulletin 91/45**

(51) Int. Cl.$^5$ : **A61K 31/19**, A61K 7/48, A61K 7/00, // (A61K31/19, 31:14)

(21) Numéro de dépôt : **89103375.5**

(22) Date de dépôt : **27.02.89**

(54) **Compositions pharmaceutiques et cosmétiques à base de peroxyde de benzoyle et de sels d'ammonium quaternaires.**

(30) Priorité : **09.03.88 FR 8803042**

(43) Date de publication de la demande : **04.10.89 Bulletin 89/40**

(45) Mention de la délivrance du brevet : **06.11.91 Bulletin 91/45**

(84) Etats contractants désignés : **AT BE CH DE FR GB IT LI NL**

(56) Documents cités :
**FR-A- 2 328 039**
**FR-A- 2 378 523**
**GB-A- 1 163 044**
**GB-A- 2 150 436**

(73) Titulaire : **L'OREAL**
**14, Rue Royale**
**F-75008 Paris (FR)**

(72) Inventeur : **Sebag, Henri**
**26, rue Erlanger**
**F-75016 Paris (FR)**
Inventeur : **Beck, Irina**
**70, Avenue G. Sachet**
**FR-93240 Villepinte (FR)**

(74) Mandataire : **Casalonga, Axel et al**
**BUREAU D.A. CASALONGA - JOSSE**
**Morassistrasse 8**
**W-8000 München 5 (DE)**

**EP 0 335 115 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet des compositions pharmaceutiques et cosmétiques à base de peroxyde de benzoyle et de sels d'ammonium quaternaires ainsi que leur utilisation, notamment pour le traitement de l'acné.

L'étiopathologie de l'acné bien que mal définie, prend son origine dans la formation d'une lésion caractéristique : le comédon. Celui-ci résulte de l'obstruction du canal pilosébacé par suite d'une dyskératinisation de la zone de l'infundibulum du canal. L'obstruction a pour effet majeur une hyperprolifération des souches résidantes cutanées qui déclenche alors une réaction de type inflammatoire de l'organisme.

Parmi les agents thérapeutiques préconisés dans le traitement de l'acné, le peroxyde de benzoyle s'est avéré depuis déjà de nombreuses années être un agent kératolytique particulièrement intéressant, présentant en outre de bonnes propriétés bactériostatiques.

L'utilisation des antibiotiques classiques dans le traitement de l'acné est également très répandue. En effet, ils possèdent une activité bactériostatique et anti-inflammatoire importante. Les antibiotiques actifs par voie orale sont très nombreux. Certains d'entre eux comme la clindamycine et surtout l'érythromycine présentent une activité par voie topique.

Pour augmenter l'activité des compositions antiacnéiques par voie topique, on a alors associé aux antibiotiques le peroxyde de benzoyle. Ce dernier a, en particulier, été associé à l'érythromycine (FR-A-2378523).

Cependant, les antibiotiques associés ou non au peroxyde de benzoyle, présentent l'inconvénient majeur lors d'une utilisation prolongée, de rendre la flore bactérienne résistante. Ils deviennent alors peu actifs lors de traitements ultérieurs. (LEYDEN J. J., J. Am. Acad. Dermatol. 8 (1) 41-45 (1983)).

En outre, cette association du peroxyde de benzoyle avec l'érythromycine s'avère instable dans le temps.

On a alors préconisé, (M. GLOOR, Arch. Dermatol. Res. 265 207-212 (1979)) pour remplacer les antibiotiques, d'utiliser des sels d'ammonium quaternaires dans le traitement topique de l'acné. En effet, certains sels d'ammonium quaternaires sont aussi actifs que les antibiotiques vis-à-vis des principales souches responsables de l'acné, sans induire de phénomène de résistance.

On a également décrit des compositions détergentes stables, utilisables éventuellement pour le nettoyage de la peau, pouvant contenir simultanément un agent de surface d'ammonium quaternaire tel que le chlorure de benzalkonium, de cétalkonium, de cétylpyridinium, de benzéthonium ou le bromure de céthéxonium et de peroxyde de benzoyle (FR-A-2328039).

Or, il s'avère que le peroxyde de benzoyle associé à l'un quelconque des sels d'ammonium quaternaires cités dans le brevet ci-dessus, se dégrade en quelques heures à la température ambiante.

La demanderesse vient de découvrir, de façon tout à fait surprenante, qu'il était possible d'obtenir des compositions stables dans le traitement de l'acné, de l'ulcère cutané, et de manière générale dans le traitement de dermatoses et désordres cutanés, en associant le peroxyde de benzoyle à certains dérivés ammonium quaternaires.

Du fait de sa bonne stabilité dans les associations conformes à l'invention, le peroxyde de benzoyle peut être utilisé à des faibles doses, avec pour avantage une amélioration de la tolérance cutanée.

Les compositions conformes à l'invention sont bien tolérées par l'organisme. Elles présentent de très bonnes propriétés antibactériennes, sans induire de phénomène de résistance des souches, elles sont kératolytiques, bactériostatiques, notamment vis-à-vis de Propionibacterium Acnes qui est l'un des principaux germes responsables de l'acné et se montrent actives dans le traitement et la réduction du nombre des comédons.

Du fait de leurs propriétés, les compositions selon l'invention sont appropriées dans le traitement des désordres cutanés et de dermatoses, telles que notamment l'acné, l'ulcère cutané.

La présente invention a donc pour objet une composition topique pharmaceutique et/ou cosmétique contenant du peroxyde de benzoyle et au moins un sel d'ammonium quaternaire tels que ceux décrits ci-dessous.

L'invention a également pour objet un procédé de traitement cosmétique mettant en oeuvre une telle association.

D'autres objets de l'invention apparaîtront à la lecture de la description et des exemples qui suivent.

La composition destinée à une application topique conforme à l'invention est essentiellement caractérisée par le fait qu'elle contient dans un milieu physiologiquement acceptable du peroxyde de benzoyle et un sel d'ammonium quaternaire choisi parmi les alkyl sulfates ou sulfonates, les aryl sulfonates et les alkyl aryl sulfonates d'ammonium quaternaires.

Les alkyl sulfates ou sulfonates, aryl sulfonates ou alkyl aryl sulfonates d'ammonium quaternaires associés au peroxyde de benzoyle, conformes à l'invention, sont en particulier des composés répondant essentiellement à la formule générale (I) suivante :

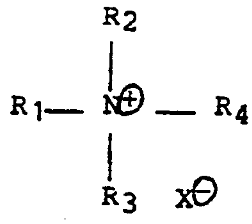

(I)

dans laquelle :

(i) $R_1$ désigne un groupement :

$$R-\left[OC_2H_3Z\right]_n-\left[OCH_2-CHOH-CH_2\right]_p-$$

dans lequel :

$0 \leq n \leq 6$ et p désigne 0 ou 1 ;

R désigne un radical alkyle, alkylcycloalkyle ou alkylaryle en $C_8$ à $C_{32}$, dont la chaîne aliphatique peut éventuellement être interrompue par un groupement éther, amide, sulfonamide ou carbamate et/ou comporter un substituant hydroxyle ;

quand n est différent de 0, Z désigne H, $CH_3$ ou $CH_2OH$ ;

quand Z désigne H ou $CH_3$, p est égal à 0 ;

quand Z désigne $CH_2OH$, p est égal à 1.

Le groupement ($OC_2H_3Z$) peut désigner l'un et/ou l'autre des enchaînements suivants :

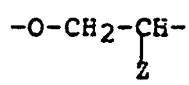 ; 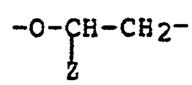

(ii) $R_2$ désigne un radical alkyle en $C_1$ à $C_{22}$ pouvant éventuellement comporter un ou plusieurs groupement(s) hydroxyle ;

(iii) $R_3$ désigne un radical alkyle en $C_1$ à $C_6$, un radical monohydroxyalkyle en $C_1$ à $C_6$, un radical polyhydroxyalkyle en $C_3$ à $C_6$, ou un radical benzyle ;

(iv) $R_4$ désigne un radical alkyle ou alkyloxy en $C_1$ à $C_{18}$ ;

(v) $R_2$ et $R_3$ peuvent former ensemble avec l'atome d'azote un hétérocycle tel que la pipéridine ou la morpholine ;

(vi) $R_1$, $R_2$ et $R_3$ peuvent former avec l'atome d'azote un cycle pyridine, méthylpyridine ou hydroxypyridine.

Dans les différents cas évoqués, $X^{\ominus}$ désigne un anion alcoyl sulfate ou sulfonate, aryl sulfonate ou alkyl aryl sulfonate.

Les anions particulièrement préférés sont choisis parmi les anions de formules suivantes :

$CH_3OSO_3^{\ominus}$ , $C_2H_5OSO_3^{\ominus}$ , $CH_3SO_3^{\ominus}$ ,

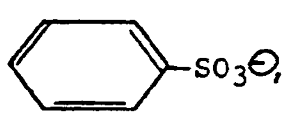 , 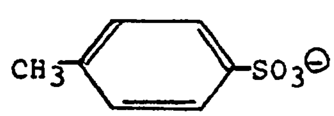 ou 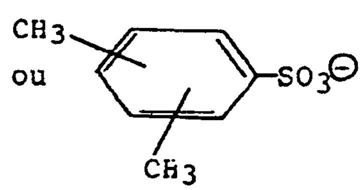

Les ammonium quaternaires particulièrement préférés sont les alkyl sulfates, sulfonates, aryl sulfonates ou alkyl aryl sulfonates de :

— triméthyl lauryl ammonium,

— diméthyl hydroxyéthyl cétyl ammonium,

— diméthyl méthoxy lauryl ammonium,

— triméthyl laurylamido propyl ammonium,
— cétyl pyridinium,
— cétyloxy pyridinium,
— diméthyl benzyl cétyl ammonium,
— diméthyl benzyl octylphénoxyéthoxyéthyl ammonium,
— diméthyl hydroxycyclohexyl cétyl ammonium,
— N-méthyl, N'cétyl imidazolium,
— méthyl lauryl morpholinium.

Les compositions peuvent se présenter sous forme de solutions, d'émulsions, de suspensions, de gels ou de dispersions, contenant au moins un composé répondant à la formule (I) dans des concentrations comprises entre 0,01 et 25% en poids, par rapport au poids total de la composition et de préférence comprises entre 0,1 et 5% en poids et du peroxyde de benzoyle dans des concentrations comprises entre 0,1 et 20% en poids par rapport au poids total de la composition et de préférence comprises entre 0,5 et 10% en poids.

Ces compositions peuvent contenir des véhicules et adjuvants physiologiquement acceptables, bien connus dans l'état de la technique. On peut, par exemple, préparer des solutions, des émulsions, des micro-suspensions ou des dispersions vésiculaires, en utilisant un ou plusieurs support(s) organique(s) acceptable(s) du point de vue physiologique, choisi(s) en plus de l'eau parmi l'éthanol, l'isopropanol, les éthers de glycol ou de polyglycols, les polyalkylène-glycols, les huiles naturelles ou de synthèse telles que les triglycérides, les alcools gras ou les esters d'acides gras.

Les compositions conformes à l'invention peuvent également renfermer des tensio-actifs moussants ou émulsionnants, des polymères tels que la cellulose et ses dérivés, la gomme de guar, les hétérobiopolysaccharides, les acides polyacryliques et leurs dérivés, la poly-β-alanine, les éthers ou esters de polyéthylèneglycols, de la silice colloïdale, des surgraissants, des émollients, des mouillants, des régulateurs de pH, des agents de pénétration, des conservateurs, des filtres solaires, des parfums, des colorants et/ou des pigments ayant pour fonction de colorer la peau ou la composition elle-même et tout autre ingrédient habituellement utilisé dans des compositions destinées à une application topique.

Bien entendu, les excipients et ingrédients qui pourraient réagir de façon indésirable avec le peroxyde de benzoyle, utilisé conformément à l'invention, doivent être exclus.

Les compositions conformes à l'invention peuvent également contenir en association des agents antiacnéiques tels que les dérivés rétinoïques, les agents antibactériens, les anti-inflammatoires, les stéroïdiens à action non hormonale, notamment la pregnénolone, et/ou des agents kératolytiques ou comédolytiques.

Les formes galéniques principalement conditionnées pour la voie topique se présentent notamment sous forme de solutions, de crèmes, de laits, de gels, de dispersions ou micro-émulsions plus ou moins épaissies, de tampons imbibés, de pommades, de sticks ou sous forme de pains de savon.

Les compositions pharmaceutiques, conformes à l'invention, du fait de leurs propriétés antibactériennes et kératolytiques notamment, peuvent être utilisées à titre de médicament dans le traitement thérapeutique de dermatoses, et en particulier de l'acné.

Un autre objet de l'invention est donc également constitué par l'utilisation des compositions pharmaceutiques pour la préparation d'un médicament destiné au traitement de dermatoses, tels qu'en particulier l'acné, les ulcères cutanés, les verrues et dyskératinisations de la peau.

Les compositions, conformes à l'invention, peuvent être utilisées pour le traitement cosmétique de la peau, notamment comme produit nettoyant et désinfectant, comédolytique, kératolytique.

Un autre objet de l'invention concerne un procédé de traitement cosmétique caractérisé par le fait qu'il consiste à appliquer sur la peau une composition conforme à l'invention en vue d'assainir ou d'épurer cette dernière.

Le traitement de l'acné peut être effectué en appliquant la composition sur les zones atteintes à raison d'une ou deux applications par jour pendant une à douze semaines.

Les exemples ci-après sont destinés à illustrer l'invention.

## EXEMPLE 1

On prépare la composition suivante :

| | | |
|---|---|---|
| - Stéarate de PEG 50 | | 4,00 g |
| - Monostéarate de glycérol | | 1,00 g |
| - Alcool stéarylique | | 1,00 g |
| - Alcool cétylique | | 1,00 g |
| - Perhydrosqualène | | 12,00 g |
| - Hydroxy éthyl cellulose | | 0,30 g |
| - Para hydroxy benzoate de méthyle | | 0,10 g |
| - Tampon | qsp | pH 6 |
| - EDTA disodique | | 0,05 g |
| - Peroxyde de benzoyle | | 5,00 g |
| - p-toluène sulfonate de dodécyl pyridinium | | 0,70 g |
| - Eau | qsp | 100,00 g |

Cette composition est utilisée sous forme de crème dermique une à deux fois par jour pendant une à douze semaines.

EXEMPLE 2

| | | |
|---|---|---|
| - Hydroxyéthyl cellulose | | 0,80 g |
| - Polysorbate 20 (sorbitan monolaurate de polyoxyéthylène (20)) | | 2,50 g |
| - Peroxyde de benzoyle | | 5,00 g |
| - Méthyl sulfate de diméthyl méthoxy lauryl ammonium | | 0,50 g |
| - Glycérine | | 3,00 g |
| - Tampon | qsp | pH 6 |
| - Eau | qsp | 100,00 g |

Cette composition est utilisée sous forme de lotion une à deux fois par jour jusqu'à la disparition des lésions acnéiques.

EXEMPLE 3

| | | |
|---|---|---|
| - Hydroxy éthyl cellulose | | 1,00 g |
| - Propylène glycol | | 3,00 g |
| - Peroxyde de benzoyle | | 10,00 g |
| - Méthyl sulfate de triméthyl lauryl ammonium | | 1,00 g |
| - Ethanol 95° | | 10,00 g |
| - Poloxamer 182 copolymère de polyoxyéthylène/polyoxypropylène de formule : 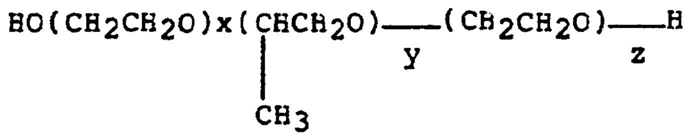 avec x=8, y=30, 2=8). | | 0,30 g |
| - EDTA disodique | | 0,05 g |
| - Tampon | qsp pH 6 | |
| - Eau | qsp | 100,00 g |

Cette composition est utilisée sous forme de gel dermique que l'on applique deux fois par jour sur les zones affectées de la peau.

## Revendications

1. Composition destinée à l'application topique, caractérisée par le fait qu'elle contient dans un milieu physiologiquement acceptable, du peroxyde de benzoyle et un sel d'ammonium quaternaire choisi parmi les alkyl sulfates ou sulfonates, les aryl sulfonates et les alkyl aryl sulfonates d'ammonium quaternaires.

2. Composition selon la revendication 1, caractérisée par le fait que le sel d'ammonium quaternaire répond à la formule générale (I) :

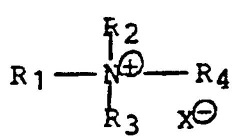

(I)

dans laquelle :

(i) $R_1$ désigne un groupement :

$$R-\left[OC_2H_3Z\right]_n-\left[OCH_2-CHOH-CH_2\right]_p-$$

dans lequel :

$0 \leq n \leq 6$ et p désigne 0 ou 1 ;

R désigne un radical alkyle, alkylcycloalkyle ou alkylaryle en $C_8$ à $C_{32}$, dont la chaîne aliphatique peut éventuellement être interrompue par un groupement éther, amide, sulfonamide ou carbamate et/ou comporter un substituant hydroxyle ;

quand n est différent de 0, Z désigne H, $CH_3$ ou $CH_2OH$ ;
quand Z désigne H ou $CH_3$, p est égal à 0 ;
quand Z désigne $CH_2OH$, p est égal à 1 ;
le groupement ($OC_2H_3Z$) peut désigner l'un et/ou l'autre des enchaînements suivants :

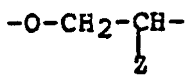 ; 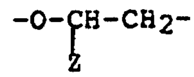

(ii) $R_2$ désigne un radical alkyle en $C_1$ à $C_{22}$ pouvant éventuellement comporter un ou plusieurs groupement(s) hydroxyle ;
(iii) $R_3$ désigne un radical alkyle en $C_1$ à $C_6$, un radical monohydroxyalkyle en $C_1$ à $C_6$, un radical polyhydroxyalkyle en $C_3$ à $C_6$, ou un radical benzyle ;
(iv) $R_4$ désigne un radical alkyle ou alkyloxy en $C_1$ à $C_{18}$ ;
(v) $R_2$ et $R_3$ peuvent former ensemble avec l'atome d'azote un hétérocycle tel que la pipéridine ou la morpholine, ou
(vi) $R_1$, $R_2$ et $R_3$ peuvent former avec l'atome d'azote un cycle pyridine, méthylpyridine ou hydroxy-pyridine;
$X^{\ominus}$ désigne un anion alkyl sulfate ou sulfonate, aryl sulfonate ou alkyl aryl sulfonate.

3. Composition selon la revendication 2, caractérisée par le fait que l'anion $X^{\ominus}$ est choisi parmi les groupements de formules suivantes :

$$CH_3OSO_3^{\ominus} \ , \ C_2H_5OSO_3^{\ominus} \ , \ CH_3SO_3^{\ominus} \ ,$$

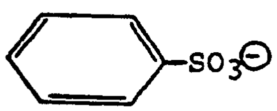 , 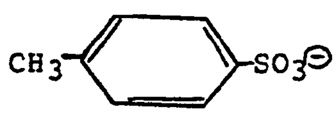 ou 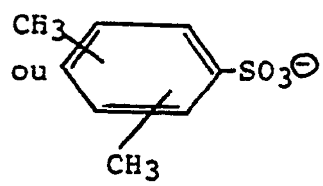

4. Composition selon l'une des revendications 1 à 3, caractérisée par le fait que l'ammonium quaternaire répondant à la formule générale (I) est choisi parmi le triméthyl lauryl ammonium, le diméthyl hydroxyéthyl cétyl ammonium, le diméthyl méthoxy lauryl ammonium, le triméthyl laurylamido propyl ammonium, le cétyl pyridinium, le cétyloxy pyridinium, le diméthyl benzyl cétyl ammonium, le diméthyl benzyl octylphénoxy éthoxyéthyl ammonium, le diméthyl hydroxycyclohexyl cétyl ammonium, le N-méthyl, N'cétyl imidazolium ou le méthyl lauryl morpholinium.

5. Composition selon l'une quelconque des revendications 1 à 4, caractérisée par le fait que le peroxyde de benzoyle est présent dans des proportions de 0,1 à 20% en poids par rapport au poids total de la composition.

6. Composition selon l'une quelconque des revendications 1 à 5, caractérisée par le fait que l'ammonium quaternaire répondant à la formule générale (I) est présent dans des proportions de 0,01 à 25% en poids par rapport au poids total de la composition.

7. Composition selon la revendication 5, caractérisée par le fait que le peroxyde de benzoyle est présent dans des proportions de 0,5 à 10% en poids par rapport au poids total de la composition.

8. Composition selon la revendication 6, caractérisée par le fait que l'ammonium quaternaire répondant à la formule générale (I) est présent dans des proportions de 0,1 à 5% en poids par rapport au poids total de la composition.

9. Composition selon l'une quelconque des revendications 1 à 8, pour son application à titre de médicament, caractérisée par le fait que le support approprié est un support pharmaceutiquement acceptable.

10. Composition selon l'une quelconque des revendications 1 à 8, caractérisée par le fait que le support approprié est un support cosmétiquement acceptable.

11. Composition selon l'une quelconque des revendications 1 à 10, caractérisée par le fait qu'elle se présente sous forme de gels, de solutions, de dispersions, d'émulsions ou de suspensions.

12. Composition selon l'une quelconque des revendications 1 à 11, caractérisée par le fait qu'elle se présente sous forme de crème, de lait, de gel, de tampon imbibé, de pommade, de stick ou sous forme de pain de savon.

13. Composition selon l'une quelconque des revendications 1 à 12, caractérisée par le fait qu'elle comprend

en outre au moins un autre agent antiacnéique.

14. Composition selon l'une quelconque des revendications 1 à 13, caractérisée par le fait qu'elle comprend en outre au moins un filtre solaire.

15. Utilisation des compositions définies selon la revendication 9, pour la préparation d'un médicament pour le traitement de dermatoses.

16. Utilisation des compositions selon la revendication 15, pour le traitement des ulcères cutanés, des verrues et dyskératinisations de la peau.

17. Utilisation des compositions définies selon la revendication 9, pour la préparation d'un médicament pour le traitement de l'acné.

18. Procédé de traitement cosmétique, caractérisé par le fait qu'il consiste à appliquer sur la peau une composition telle que définie dans la revendication 10, en vue de nettoyer et d'assainir la peau.

**Patentansprüche**

1. Zusammensetzung zur topischen Anwendung, dadurch **gekennzeichnet**, dass sie in einem physiologisch akzeptablen Milieu Benzoylperoxid und ein quartäres Ammoniumsalz umfasst, ausgewählt aus den quartären Ammoniumsalzen von Alkylsulfaten oder -sulfonaten, Arylsulfonaten und den Alkylarylsulfonaten.

2. Zusammensetzung nach Anspruch 1, dadurch **gekennzeichnet**, dass das quartäre Ammoniumsalz durch die folgende allgemeine Formel (I) dargestellt ist :

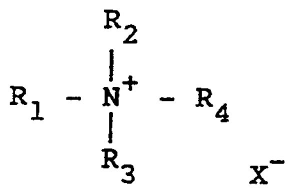

(I)

worin bedeuten :

(i) $R_1$ eine Gruppe

$$R-[OC_2H_3Z]_n-[OCH_2-CHOH-CH_2]_p—$$

worin bedeuten :

$0 \leq n \leq 6$ und p bedeutet 0 oder 1 ;

R bedeutet ein Alkylradikal, ein Alkylcycloalkylradikal oder Alkylarylradikal mit 8 bis 32 C-Atomen, wobei die aliphatische Kette gegebenenfalls durch eine Ether-, Amid-, Sulfonamid- oder Carbamatgruppe unterbrochen sein kann und/oder einen Hydrokylsubstituenten tragen kann ;

wenn n von 0 unterschiedlich ist, bedeutet Z H, $CH_3$ oder $CH_2OH$ ;

wenn Z H oder $CH_3$ bedeutet, ist p gleich 0 ;

wenn Z $CH_2OH$ bedeutet, ist p gleich 1 ;

die Gruppe ($OC_2H_3Z$) kann die eine oder die andere der nachfolgenden Verknüpfungen aufweisen :

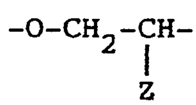 ; 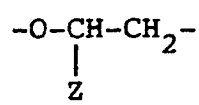

(ii) $R_2$ bedeutet ein $C_{1-22}$-Alkylradikal, welches gegebenenfalls eine oder mehrere Hydrokylgruppen umfassen kann ;

(iii) $R_3$ bedeutet ein $C_{1-6}$-Alkylradikal, ein $C_{1-6}$-Monohydrokyalkylradikal, ein $C_{3-6}$-Polyhydrokyalkylradikal oder ein Benzylradikal ;

(iv) $R_4$ bedeutet ein Alkyl- oder Alkyloxyradikal mit 1 bis 18 C-Atomen ;

(v) $R_2$ und $R_3$ können zusammen mit dem Stickstoffatom einen Heterozyklus, wie das Piperidin oder Morpholin bilden ; oder

(vi) $R_1$, $R_2$ und $R_3$ können mit dem Stickstoffatom einen Pyridin-, Methylpyridin- oder Hydrokypyridinring bilden ;

$X^-$ bedeutet ein Alkylsulfat- oder -sulfonat-, Arylsulfonat- oder Alkylarylsulfonat-anion.

3. Zusammensetzung nach Anspruch 2, dadurch **gekennzeichnet**, dass das Anion $X^-$ aus den Gruppen mit den folgenden Formeln ausgewählt ist :

$CH_3OSO_3^-$, $C_2H_5OSO_3^-$, $CH_3SO_3^-$,

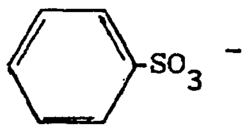
,
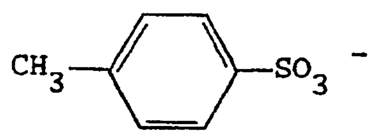

oder

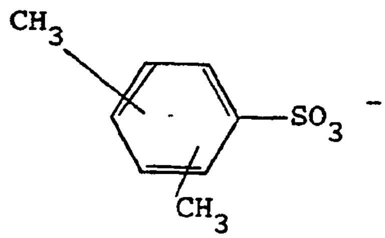

4. Zusammensetzung nach einem der Ansprüche 1 bis 3, dadurch **gekennzeichnet**, dass das quartäre Ammoniumsalz, welches durch die allgemeine Formel (I) dargestellt ist, ausgewählt ist aus Trimethyl-laurylammonium, Dimethyl-hydroxyethyl-cetylammonium, Dimethyl-methoxy-laurylammonium, Trimethyl-laurylamidopropylammonium, Cetylpyridinium, Cetyloxypyridinium, Dimethyl-benzyl-cetylammonium, Dimethyl-benzyl-octylphenory-ethoxyethylammonium, Dimethyl-hydroxycyclohexyl-cetylammonium, N-Methyl-, N-Cetylimidazolium- oder Methyllauryl-morpholinium.

5. Zusammensetzung nach einem der Ansprüche 1 bis 4, dadurch **gekennzeichnet**, dass das Benzoylperoxid in einer Menge von 0,1 bis 20 Gew.%, bezogen auf das Gesamtgewicht der Zusammensetzung, vorhanden ist.

6. Zusammensetzung nach einem der Ansprüche 1 bis 5, dadurch **gekennzeichnet**, dass das quartäre Ammoniumsalz der allgemeinen Formel (I) in einer Menge von 0,01 bis 25 Gew.%, bezogen auf das Gesamtgewicht der Zusammensetzung, vorhanden ist.

7. Zusammensetzung nach Anspruch 5, dadurch **gekennzeichnet**, dass das Benzoylperoxid in einer Menge von 0,5 bis 10 Gew.%, bezogen auf das Gesamtgewicht der Zusammensetzung, vorhanden ist.

8. Zusammensetzung nach Anspruch 6, dadurch **gekennzeichnet**, dass das quartäre Ammoniumsalz der allgemeinen Formel (I) in einer Menge von 0,1 bis 5 Gew.%, bezogen auf das Gesamtgewicht der Zusammensetzung, vorhanden ist.

9. Zusammensetzung nach einem der Ansprüche 1 bis 8 zur Anwendung als Medikament, dadurch **gekennzeichnet**, dass der geeignete Träger ein pharmazeutisch akzeptabler Träger ist.

10. Zusammensetzung nach einem der Ansprüche 1 bis 8, dadurch **gekennzeichnet**, dass der geeignete Träger ein kosmetisch akzeptabler Träger ist.

11. Zusammensetzung nach einem der Ansprüche 1 bis 10, dadurch **gekennzeichnet**, dass sie in Form von Gelen, Lösungen, Dispersionen, Emulsionen oder Suspensionen vorliegt.

12. Zusammensetzung nach einem der Ansprüche 1 bis 11, dadurch **gekennzeichnet**, dass sie in Form einer Creme, einer Milch, eines Gels, eines imprägnierten Tupfers, einer Pommade, eines Stiftes oder in Form eines Stückes Seife vorhanden ist.

13. Zusammensetzung nach einem der Ansprüche 1 bis 12, dadurch **gekennzeichnet**, dass sie zusätzlich zumindest ein anderes Anti-Aknemittel enthält.

14. Zusammensetzung nach einem der Ansprüche 1 bis 13, dadurch **gekennzeichnet**, dass sie zusätzlich zumindest einen Sonnenfilter enthält.

15. Verwendung der Zusammensetzungen nach Anspruch 9 zur Herstellung eines Medikamentes zur Behandlung von Dermatosen.

16. Verwendung der Zusammensetzungen nach Anspruch 15 zur Behandlung von ulzeröser Haut, von Warzen und Dyskeratose der Haut.

17. Verwendung der Zusammensetzungen nach Anspruch 9 zur Herstellung eines Medikamentes zur Behandlung von Akne.

18. Verfahren zur kosmetischen Behandlung, **gekennzeichnet** durch Auftragung einer Zusammensetzung, wie sie in Anspruch 10 definiert ist, auf die Haut, um die Haut zu reinigen und zu säubern.

## Claims

1. Composition intended for topical application, characterised in that it contains, in a physiologically acceptable medium, benzoyl peroxide and a quaternary ammonium salt selected from quaternary ammonium alkyl sulphates or alkylsulphonates, arylsulphonates and alkylarylsulphonates.

2. Composition according to Claim 1, characterised in that the quaternary ammonium salt corresponds to the general formula (I) :

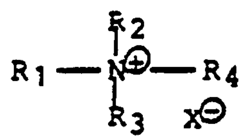

(I)

in which :

(i) $R_1$ denotes a group :

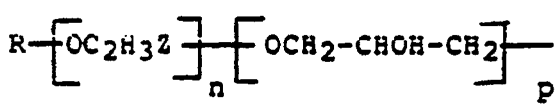

in which :

$0 \leq n \leq 6$ and p denotes 0 or 1 ;

R denotes a $C_8$ to $C_{32}$ alkyl, alkylcycloalkyl or alkylaryl radical in which the aliphatic chain can optionally be interrupted by an ether, amide, sulphonamide or carbamate group and/or contain a hydroxyl substituent ;

when n is other than 0, Z denotes H, $CH_3$ or $CH_2OH$ ;

when Z denotes H or $CH_3$, p is equal to 0 ;

when Z denotes $CH_2OH$, p is equal to 1 ;

the group $(OC_2H_3Z)$ can denote either or both of the following arrangements :

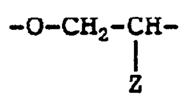 ; 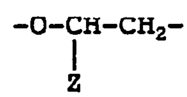

(ii) $R_2$ denotes a $C_1$ to $C_{22}$ alkyl radical which can optionally contain one or more hydroxyl group(s) ;

(iii) $R_3$ denotes a $C_1$ to $C_6$ alkyl radical, a $C_1$ to $C_6$ monohydroxyalkyl radical, a $C_3$ to $C_6$ polyhydroxyalkyl radical or a benzyl radical ;

(iv) $R_4$ denotes a $C_1$ to $C_{18}$ alkyl or alkoxy radical ;

(v) $R_2$ and $R_3$, together with the nitrogen atom, can form a heterocycle such as piperidine or morpholine, or

(vi) $R_1$, $R_2$ and $R_3$, with the nitrogen atom, can form a pyridine, methylpyridine or hydroxyridine ring ;

$X^{\ominus}$ denotes an alkyl sulphate or alkylsulphonate, arylsulphonate or alkylarylsulphonate anion.

3. Composition according to Claim 2, characterised in that the anion $X^{\ominus}$ is selected from groups of the following formulae :

$CH_3OSO3^{\ominus}$, $C_2H_5OSO_3^{\ominus}$, $CH_3SO_3^{\ominus}$,

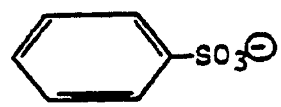 , 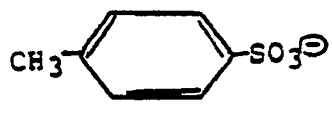 or 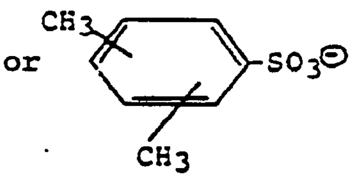

4. Composition according to one of Claims 1 to 3, characterised in that the quaternary ammonium compound corresponding to the general formula (I) is selected from trimethyllaurylammonium, dimethylhydroxyethylcetylammonium, dimethylmethoxylaurylammonium, trimethyllaurylamidopropylammonium, cetylpyridinium, cetyloxypyridinium, dimethylbenzylcetylammonium, dimethylbenzyloctylphenoxyethoxyethylammonium, dimethylhydroxycyclohexylcetylammonium, N-methyl-N'-cetylimidazolium or methyllaurylmorpholinium compounds.

5. Composition according to any one of Claims 1 to 4, characterised in that the benzoyl peroxide is present in proportions of 0.1 to 20% by weight relative to the total weight of the composition.

6. Composition according to any one of Claims 1 to 5, characterised in that the quaternary ammonium compound corresponding to the general formula (I) is present in proportions of 0.01 to 25% by weight relative to the total weight of the composition.

7. Composition according to Claim 5, characterised in that the benzoyl peroxide is present in proportions of 0.5 to 10% by weight relative to the total weight of the composition.

8. Composition according to Claim 6, characterised in that the quaternary ammonium compound corresponding to the general formula (I) is present in proportions of 0.1 to 5% by weight relative to the total weight of the composition.

9. Composition according to any one of Claims 1 to 8, for its application as a medicinal product, characterised in that the appropriate vehicle is a pharmaceutically acceptable vehicle.

10. Composition according to any one of Claims 1 to 8, characterised in that the appropriate vehicle is a cosmetically acceptable vehicle.

11. Composition according to any one of Claims 1 to 10, characterised in that it takes the form of gels, solutions, dispersions, emulsions or suspensions.

12. Composition according to any one of Claims 1 to 11, characterised in that it takes the form of a cream, milk, gel, impregnated pad, ointment or stick, or the form of a cake of soap.

13. Composition according to any one of Claims 1 to 12, characterised in that it comprises, in addition, at least one other anti-acne agent.

14. Composition according to any one of Claims 1 to 13, characterised in that it comprises, in addition, at least one sunscreen agent.

15. Use of the compositions defined according to Claim 9, for the preparation of a medicinal product for the treatment of dermatoses.

16. Use of the compositions according to Claim 15, for the treatment of skin ulcers, warts and dyskeratinisations of the skin.

17. Use of the compositions defined according to Claim 9, for the preparation of a medicinal product for the treatment of acne.

18. Cosmetic treatment process, characterised in that it consists in applying to the skin a composition as defined in Claim 10, for the purpose of cleansing and decontaminating the skin.